# EUROPEAN PATENT SPECIFICATION

(11) **EP 3 512 603 B1**
(45) Date of publication and mention of the grant of the patent: **12.02.2020**
(21) Application number: 17780136.2
(22) Date of filing: 11.10.2017
(51) Int. Cl.: A61Q 5/06, A61K 8/04, B05B 7/04, B05B 1/34, B65D 83/62, B65D 83/14

(54) **COMPRESSED HAIR SPRAY**
KOMPRIMIERTES HAARSPRAY
LAQUE SOUS PRESSION POUR CHEVEUX

(30) Priority: 19.10.2016 EP 16194550
(43) Date of publication of application: 24.07.2019
(73) Proprietor: Unilever PLC, London, Greater London EC4Y 0DY (GB); Unilever N.V., 3013 AL Rotterdam (NL)
(72) Inventor: BROWN, Gillian, Wirral Merseyside CH63 3JW (GB); FRANKLIN, Laura, Wirral Merseyside CH63 3JW (GB); GRIFFITHS, Llyr, Glyndwr, Wirral Merseyside CH63 3JW (GB); JOINSON, Leslie, Joseph, Luke, Wirral Merseyside CH63 3JW (GB); SMITH, Jonathan, Douglas, William, Wirral Merseyside CH63 3JW (GB); SMITH, Paul, James, Wirral Merseyside CH63 3JW (GB)
(74) Representative: Mathai, Neenu Grace
(86) International application number: PCT/EP2017/075918
(87) International publication number: WO 2018/073066

(56) References cited:
- US-A- 4 940 171
- US-A1- 2014 302 101
- US-A1- 2014 348 770
- Anonymous: "ACTUATORS FOR AEROSOL VALVES", , 30 September 2015 (2015-09-30), XP055323272, Retrieved from the Internet: URL:http://www.coster.com/en_GB/Download/C atalogue/Actuators-Section.pdf [retrieved on 2016-11-25]
- Anonymous: "Aerosol Valves", , 30 September 2002 (2002-09-30), XP055323276, Retrieved from the Internet: URL:http://www.coster.com/en_GB/Download/C atalogue/Valves-Section.pdf [retrieved on 2016-11-25]
- Verville: "Introduction to Aerosol Valve Technology", , 14 March 2007 (2007-03-14), XP55420614, Retrieved from the Internet: URL:http://www.southernaerosol.com/Power Point/Powerpoint pdfs/SATA_Valve_Technology.pdf [retrieved on 2017-10-31]

## Description

### Field of invention

The present invention relates to a "compressed" aerosol hairspray product provided in a can having reduced amount of hair styling composition yet providing the same number of uses as a non-compressed product.

### Background and prior art

Hairstyling products such as hairsprays are used for achieving different hairstyles and for holding hair strands in place for a period of time. Typically, hairsprays comprise film-forming polymers, which when applied to keratin-containing fibres, such as human hair, form fibre-fibre welds. These welds 'glue' the fibres together and hence impart hold to the hairstyle.

Aerosol hairspray products usually comprise a pressure- resistant container, a nozzle, a propellant, and a hairstyling formulation. A hairspray composition is normally ejected from such products via aerosol-forming nozzle. See, for example, US2009/0104138A1.

Environmental sustainability has become a matter of growing interest in aerosol applications, and concentrates have the potential to benefit both manufacturers and the environment. Aerosol concentrates offer manufacturers cost advantages in terms of reduced packaging (both primary and secondary), reduced propellant use, reduced shipping costs, and the like. The environment is benefited by a product that, compared to conventional aerosols, emits less propellant (e.g., volatile organic compound (VOC)), generates less packaging waste and, from manufacture through disposal, has a reduced carbon footprint.

It is, therefore, desirable to reduce the size and volume of pressurised products, such as hairsprays. Such products reduce the amount of hair spray composition per consumer use without impacting performance, thus maintaining the same number of consumer uses per can.

GB 2,299,507 A (Unilever) discloses low flow rate propellant driven antiperspirant compositions comprising less than 60% by weight of a propellant and an initial spray rate of no more than 0.5 g/s.

EP 674,899 B1 (Unilever) discloses concentrated deodorant compositions comprising propellant, most preferably at 30-60% by weight of the composition, and having a discharge valve adapted to allow the composition to be sprayed at an initial spray rate of less than 0.3 g/s.

US2015/0004200 (The Procter & Gamble Company) discloses an aerosol hairspray product with a pressurisable container containing an aqueous, substantially alcohol free hairstyling formulation. A spraying device is attached to the container for dispensing the hairstyling formulation and comprises a valve and a nozzle, wherein the valve comprises a valve body, a stem and a spring means. The valve body houses an insert having an insert orifice and at least two channels, which are tangentially disposed about the insert orifice. The valve body also comprises at least two vapour taps, and the insert orifice is capable of being in liquid communication with the hairstyling formulation in the reservoir.

Consumer habits and perceptions can be exceedingly difficult to change. If a product does not fit conventional spray habits, consumer acceptance of that product may be difficult, no matter how good the product. Conversely, a product that matches consumer behaviour but does not deliver on efficacy and sensory requirements is unlikely to be commercially accepted. When providing aerosol products in concentrated form, reference need be made to the manner in which the aerosol is conventionally used. A typical spray period for a hair spray product, by which is meant the time during which an actuator is actively engaged to dispense product, is typically on the order of five seconds.

Producing an acceptable aerosol concentrate requires more than simply changing the level of active, since irrespective of active level, consumers are likely to stick to their conventional application habits. Minimizing spray rate is another approach to concentrates. Typical spray rates for hair spray aerosols are of the order of 0.5 g/s to 1.5 g/s, but lower rates may be desirable for concentrated aerosols. Minimizing spray rate theoretically requires little or no disruption to consumer behavior, because the same dose can then be applied in approximately the same time, despite the aerosol being more concentrated. However, reducing spray rate presents numerous issues for product engineers. As a practical matter, reducing spray rate can exacerbate nozzle clogging and increase spluttering. Additionally, reduced spray rates present challenges in terms of providing compositions that provide acceptable sensory properties, given the confines of consumer application habits and typical application spray periods. Reduced spray rate can also result in reduced product efficacy if insufficient active is dispensed. Therefore, compression not only requires reduced spray rate but also changes to the formulation to ensure that the compressed product is acceptable to the consumer.

One method of achieving compression of spray products is to reduce the spray rate. If the spray rate is reduced, it follows that the concentration of the resin in the formulation should be doubled to compensate. However, doubling the resin in the formulation can lead to significant challenges in terms of stability because there is too much solid content. In addition it can lead to clogging issues in the valve, insert and actuator orifice. These all negatively impact product performance and integrity.

There remains a need for concentrated aerosol hair spray products that provide a combination of desirable sensory properties and dispensing properties, without compromising product efficacy. Such products reduce the amount of hair spray composition per consumer use without impacting performance, thus maintaining the same number of consumer uses per can.

We have now found that a specially developed valve can control the spray rate to achieve compression, in combination with an optimised insert, to achieve the correct spray characteristics of a hair styling formulation. Surprisingly, there is no need to double the resin concentration.

### Definition of the invention

In a first aspect of the invention there is provided an aerosol hairspray product, which comprises:
a) a pressurisable container comprising
   i) a container wall which encloses a reservoir for storing a hairstyling formulation, and
   ii) a propellant;
b) a hairstyling formulation comprising:
   (iii) from 20 wt % to 75 wt % water, by total weight of the hairstyling formulation and propellant; and
   (iv) from 1.0 wt % to 15 wt % hairstyling polymer, by total weight of the hairstyling formulation and propellant; and
c) a spraying device attached to the container for dispensing the hairstyling formulation from the reservoir of the container, which comprises a valve and an actuator,
wherein the valve comprises a valve body, said valve body comprising a stem and a spring means; and wherein the valve comprises a restricted tail piece (RTP) of diameter 0.1 mm to 2.5 mm, and a vapour phase tap (VPT) of diameter 0.1 mm to 1.5 mm;
wherein the actuator comprises a main spray channel and communicates with an insert, said insert comprising an exit orifice and from 1 to 8 sub-spray channels; wherein said channels are tangentially disposed about the exit orifice;
wherein the exit orifice is capable of being in liquid communication with the hairstyling formulation in the reservoir;
characterised in that the exit orifice has a diameter of from 0.01 to 2 mm; and an orifice thickness of from 0.1 to 1.5 mm;
and wherein the sub-spray channels have a height of from 0.1 to 0.8 mm and a length of from 0.1 to 0.5mm.

In a second aspect of the invention there is provided a method of treating hair with a hairspray product of the aspect of the invention, comprising the step of applying to the hair a hair styling formulation as defined in the first aspect of the invention, using a hair spray product of the first aspect of the invention.

### Brief Description of the Figures

Embodiments of the invention will now be described with reference to the following non-limiting drawings in which:
Figure 1 is a perspective view of a hairspray product in accordance with the invention.
Figure 2 is a cross section view of a spraying device (4) used in the hairspray product of the invention.
Figure 3 is a cross section view of a valve (5) used in the hairspray product of the invention, where the valve is installed in the container (seated in a valve cup (16)).
Figure 4 (a) is a cross section view of an insert (6) as used in the hairspray product of the invention.
Figure 4 (b) is a cross sectional view of an insert (6) for use in the hairspray of the invention.

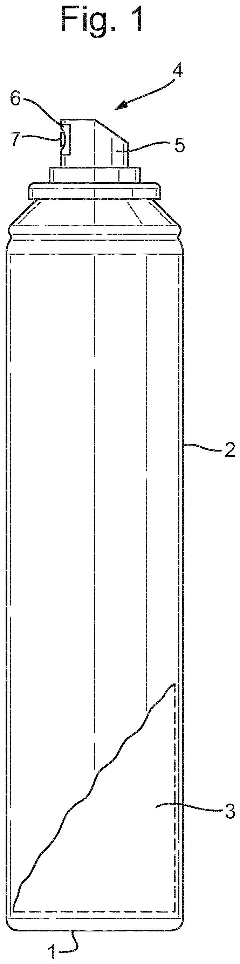

Figure 1 is a perspective view of a hairspray product comprising a pressurisable container (1) having a container wall (2) and a reservoir (3); with a spraying device (4) comprising an actuator (5) having an insert (6) with an exit orifice (7).

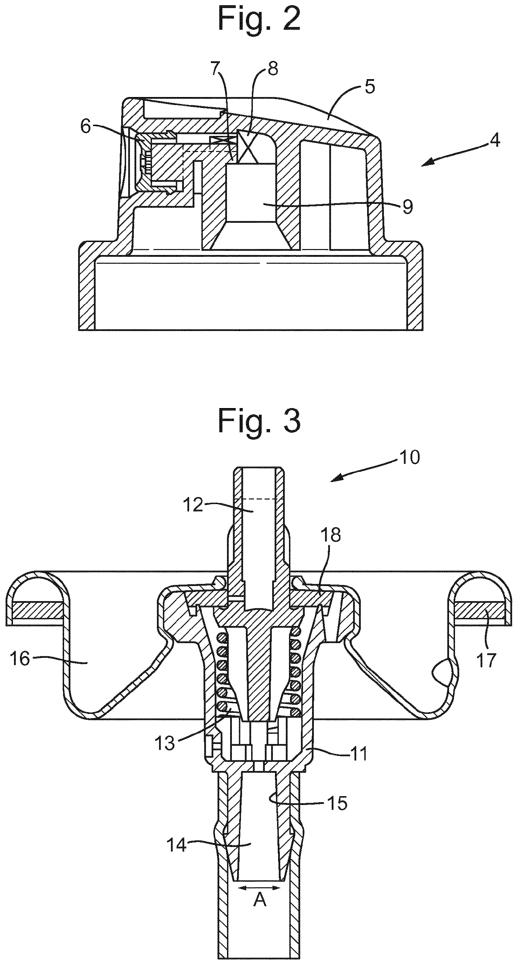

Figure 2 is a cross section view of a spraying device (4) comprising an actuator (5) having a main spray channel (8) and an insert (6) with an exit orifice (7). A stem socket is also shown.

Figure 3 is a cross sectional view of a valve (10) used in the hairspray product of the invention, where the valve is installed in the container, seated in a valve cup (16). The valve (10) comprises a valve body (11), said valve body comprising a stem (12) and a spring means (13); and wherein the valve has a restricted tail piece (RTP) (14) with a diameter indicated by a double ended arrow "A", and a vapour phase tap (VPT) (15). The valve cup (16) comprises an outer gasket (17) and an inner gasket (18).

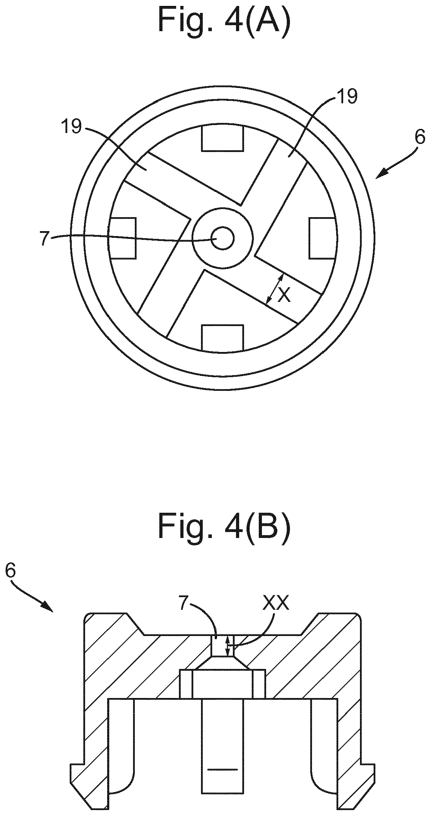

Figure 4a is a cross section view of an insert (6) comprising an exit orifice (7) and four sub-spray channels (19); wherein said channels are tangentially disposed about the exit orifice (7). Double ended arrow "X" indicates the length of the sub-spray channel as defined in claim 1.

Figure 4b is a cross section view of an insert (6) showing the exit orifice (7). Double ended arrow "XX" indicates the orifice thickness as defined in claim 1.

### General description of the Invention

### The aerosol hairspray product

The product relates to an aerosol hairspray product comprising: a container comprising a reservoir for a hairstyling formulation and a liquefied gas propellant, and a spraying device attached to the container.

In at least one embodiment, the product comprises from 50% to 65% hairstyling formulation, or from 55% to 60% hairstyling formulation, by total weight of the hairstyling formulation and propellant.

In at least one embodiment, the product comprises from 35% to 50% propellant, or from 40% to 45% propellant, by total weight of the hairstyling formulation and propellant.

As used herein, unless otherwise stated, details of the hairstyling formulation refers to the formulation before it is placed into the container whereupon it will mix to an extent with the propellant since the propellant is a liquefied gas propellant. The same is true where details of the propellant are mentioned, unless otherwise stated.

The product is an aerosol hairspray product and thus does not include mousse products or any pump spray products.

Conventional spray rates are typically in the range of from 0.5 to 1.5 g/sec. The product of the invention delivers 80 to 20 % of this range, preferably 70 to 30 %, most preferably from 60 to 40 % of this spray rate. This reduction in spray rate without loss of performance is one of the advantages of this invention. Therefore, the aerosol hairspray product sprays at a delivery rate of from 80 to 20 %, preferably from 70 to 30 %, most preferably from 60 to 40 % of 0.5 to 1.5 g/sec.

### The Pressurisable Container

The pressurisable container comprises a container wall which encloses a reservoir for storing a hairstyling formulation, and a propellant.

The container wall may be predominantly straight, curved or tapered.

The container can be any size, but is preferably a "compressed" size or travel size.

The container preferably has a volume (so-called overfill capacity) of from 200 to 300 ml, more preferably from 220 to 280 ml, most preferably from 230 to 260 ml.

The container preferably has a diameter of from 35 to 50 mm, more preferably from 40 to 45 mm.

The container preferably has a height of from 80 to 220 mm, preferably from 90 to 180 mm, most preferably from 110 to 150 mm.

### The propellant

The hairspray composition further comprises a propellant. Preferred propellants are selected from hydrofluorocarbons (for example hydrofluorocarbon 152A), dimethylether, hydrocarbons such as propane, isobutane, butane and mixtures thereof, and compressed gasses such as nitrogen and carbon dioxide and mixtures thereof.

### The hairstyling formulation

The product comprises a hairstyling formulation comprising a hair styling polymer and a neutraliser in an aqueous composition. It is well known in the art that alcoholic based composition can lead to perceived consumer negative performance such as hair dryness. These negatives are not experienced with aqueous compositions.

### The hair styling polymer

The hairstyling polymer is present in an amount of from 1.0 to 10 wt %, preferably from 1.0 to 8.0 wt %, most preferably from 2 to 7 wt % by total weight of the hairstyling formulation and propellant.

The hairstyling polymer may be selected from a VA/Crotonates/Vinyl Neodecanoate Copolymer, Polyurethane-14 (and) AMP-Acrylates Copolymer (for example, DynamX available from AzkoNoble), Butyl Ester of PVM/MA Copolymer (for example, Gantrez available from Ashland), Polyester-5, Octylacrylamide/Acrylates/Butylaminoethyl Methacrylate Copolymer, imidized isobutylene/maleic anhydride copolymer (for example, Aquaflex™ FX-64 available from Ashland), Polyvinylpyrrolidone, Acrylates/Hydroxyesters Acrylates Copolymer (for example, Acudyne 180 available from DOW), Polyurethane-6, Acrylates/Octylacrylamide Copolymer and Acrylates Copolymer (for example, Balance CR available from AkzoNoble and Luvimer 100 P available from BASF), and more preferably from VA/Crotonates/Vinyl Neodecanoate Copolymer (for example, Resyn 28-2930 available from AkzoNoble) and Octylacrylamide/Acrylates/Butylaminoethyl Methacrylate Copolymer (for example, Amphomer available from AkzoNoble).

Preferably the styling resin comprises VA/Crotonates/Vinyl Neodecanoate Copolymer (for example, Resyn 28-2930 available from AkzoNoble) and Octylacrylamide/Acrylates/Butylaminoethyl Methacrylate Copolymer (for example, Amphomer available from AkzoNoble).

### The neutralizer

The neutralizer provides an alkaline base.
The neutralizer is preferably selected from potassium hydroxide, sodium hydroxide, triisopropanolamine, 2-aminobutanol, 2-aminomethyl propanol, aminoethylpropandiol, dimethyl stearamine, sodium silicate, tetrahydroxypropyl ethylenediamine, ammonia, triethanolamine, trimethylamine, aminomerhylpropandiol, and more preferably 2-aminomethyl propanol, dimethyl stearamine and 2-aminobutanol and mixtures thereof.

### Aqueous Composition

The water is present in an amount of from 20 wt % to 75 wt % by total weight of the hairstyling formulation and propellant, preferably from 30 wt % to 70 wt %, more preferably from 35 wt % to 60 wt %, most preferably from 35 wt % to 55 wt %, by total weight of the hairstyling formulation and propellant.

The hairstyling formulation preferably contains less than 20 wt % alcohol, more preferably less than 10 wt %, even more preferably 5 wt % and most preferably is substantially free of alcohol.

### Additional ingredients

The formulation may also comprise other adjuncts commonly used in hair sprays. Preferably perfume, silicone (for example phenyl trimethicone, cyclopentylsiloxane, PEG/PPG-20/15 Dimethicone), emotives, corrosion inhibitors, emollients, UV absorbers (for example benzopheonone-4, ethyl hexyl methoxy cinnamate) and mixtures thereof.

### The Spraying Device

Attached to the container is a spraying device for spraying the hairstyling formulation onto a substrate.

### Valve

The spraying device comprises a valve.

The valve comprises a valve body, said valve body comprising a stem and a spring means.

The valve comprises a restricted tail piece (RTP) of diameter 0.1 mm to 2.5 mm, preferably 0.2 to 1.5 mm, most preferably 0.3 to 1.0 mm.

The valve comprises a vapour phase tap (VPT) of diameter 0.1 mm to 1.5 mm, preferably 0.2 to 1.0 mm, most preferably 0.3 to 0.8 mm.

When installed in the container, the valve is preferably seated in a valve cup (16). The valve cup (16) preferably comprises an outer gasket (17), which enables connection to the can by crimping onto a can bead; and an inner gasket (18). The inner gasket controls the release product when the actuator is pressed.

### Actuator

The spraying device comprises an actuator. The actuator typically sits over the stem.

The actuator comprises a main spray channel and communicates with an insert.

### Insert

The insert comprises an exit orifice and from 1 to 8 sub-spray channels, preferably 4 to 6 sub-spray channels, most preferably 6 sub-spray channels; wherein said channels are tangentially disposed about the exit orifice.

Suitable inserts are available from, for example, Coster.

The insert controls the cone angle. A wide cone angle is preferred whilst maintaining the desirable particle size distribution.

### Channels

The sub-spray channels have a height of from 0.1 to 0.8, preferably from 0.3 to 0.7 mm and a length of from 0.1 to 0.5, preferably from 0.25 to 0.35 mm.

### Exit Orifice

The exit orifice is capable of being in liquid communication with the hairstyling formulation in the reservoir.

The exit orifice has a diameter of from 0.01 to 2 mm, most preferably 0.5 mm; and an orifice thickness of from 0.1 to 1.5 mm, preferably from 0.3 to 1.0 mm;
The orifice may be straight or conical in shape.

### Examples

A hair spray in accordance with the invention and a comparative hair spray are given in Table 1 below.

**Table 1:**

| | **Comparative Hair Spray A** | **Inventive Hair Spray 1** |
|---|---|---|
| **Spraying Device** | | |
| Valve RTP | 0.33 mm | 0.8mm |
| Valve VPT | None | 0.5mm |
| Valve STEM | 0.33 mm | 0.3mm |
| Actuator (type & orifice) | Aptar 0.64 mm | Coster 0.5 mm |
| Actuator No. of channels | n/a | 6 |
| | | |
| **Spray rate** | 100% | 50% |
| | | |

| **Hairstyling Formulation** | | |
|---|---|---|
| Total hairstyling Polymer | 4-5% | 5.5% |
| Water | 53% | 53% |
| Propellant | 40% | 40% |
| Minors (fragrance, pH modifiers) | 2 - 3% | 1.5% |

## Claims

1. An aerosol hairspray product, which comprises:
a) a pressurisable container comprising
i) a container wall which encloses a reservoir for storing a hairstyling formulation, and
ii) a propellant;
b) a hairstyling formulation comprising:
(iii) from 20 wt % to 75 wt % water, by total weight of the hairstyling formulation and propellant; and
(iv) from 1.0 wt % to 15 wt % hairstyling polymer, by total weight of the hairstyling formulation and propellant; and
c) a spraying device attached to the container for dispensing the hairstyling formulation from the reservoir of the container, which comprises a valve and an actuator,
wherein the valve comprises a valve body, said valve body comprising a stem and a spring means; and wherein the valve comprises a restricted tail piece (RTP) of diameter 0.1 mm to 2.5 mm, and a vapour phase tap (VPT) of diameter 0.1 mm to 1.5 mm;
wherein the actuator comprises a main spray channel and communicates with an insert, said insert comprising an exit orifice and from 1 to 8 sub-spray channels; wherein said channels are tangentially disposed about the exit orifice;
wherein the exit orifice is capable of being in liquid communication with the hairstyling formulation in the reservoir;
**characterised in that** the exit orifice has a diameter of from 0.01 to 2 mm; and an orifice thickness of from 0.1 to 1.5 mm;
and wherein the sub-spray channels have a height of from 0.1 to 0.8 mm and a length of from 0.1 to 0.5mm.

2. An aerosol hairspray product as claimed in claim 1, comprises less than 20 wt % alcohol, by total weight of the hairstyling formulation and propellant.

3. An aerosol hairspray product as claimed in claim 1 or claim 2, wherein the formulation contains less than 5 wt% alcohol.

4. An aerosol hairspray product as claimed in any preceding claim, wherein the hairstyling polymer is present in an amount of from 1.0 to 10 wt by total weight of the hairstyling formulation and propellant.

5. An aerosol hairspray product as claimed in any preceding claim, wherein the styling polymer is selected from a VA/Crotonates/Vinyl Neodecanoate Copolymer, Polyurethane-14 (and) AMP-Acrylates Copolymer, Butyl Ester of PVM/MA Copolymer, Polyester-5, Octylacrylamide/Acrylates/Butylaminoethyl Methacrylate Copolymer, Acrylates/Hydroxyesters Acrylates Copolymer, Polyurethane-6, Acrylates/Octylacrylamide Copolymer and Acrylates Copolymer, and more preferably from VA/Crotonates/Vinyl Neodecanoate Copolymer and Octylacrylamide/Acrylates/Butylaminoethyl Methacrylate Copolymer.

6. An aerosol hairspray product as claimed in any preceding claim, wherein the formulation comprises an alkaline base.

7. An aerosol hairspray product as claimed in any preceding claim, wherein the RTP has a diameter of from 0.2 to 1.5 mm, preferably from 0.3 to 1.0 mm.

8. An aerosol hairspray product as claimed in any preceding claim, wherein the VPT has a diameter of from is from 0.2 to 1.0 mm, preferably from 0.3 to 0.8 mm.

9. An aerosol hairspray product as claimed in any preceding claim, wherein the container has a volume of from 200 to 300 ml.

10. An aerosol hairspray product as claimed in any preceding claim, wherein the exit orifice comprises 6 sub-spray channels.

11. An aerosol hairspray product as claimed in any preceding claim, wherein the sub-spray channels have a height of from 0.3 to 0.7 mm.

12. An aerosol hairspray product as claimed in any preceding claim, wherein the sub-spray channels have a length of from 0.25 to 0.35 mm.

13. An aerosol hairspray product as claimed in any preceding claim, wherein the exit orifice has an orifice thickness of from 0.1 to 1.5 mm.

14. A method of treating hair with a hairspray product as defined in any one of claims 1 to 13, comprising the step of applying to the hair a hair styling formulation as defined in any one of claims 1 to 6, using a hair spray product as defined in any of claims 1 to 13.

## Patentansprüche

1. Aerosolhaarsprayprodukt, das Folgendes umfasst:
a) ein mit Druck beaufschlagbares Gefäß, das Folgendes umfasst:
i) eine Gefäßwand, die einen Behälter zum Bevorraten einer Haarformgebungsrezeptur umschließt, und
ii) ein Treibmittel;
b) eine Haarformgebungsrezeptur, die Folgendes umfasst:
(iii) 20 Gew.-% bis 75 Gew.-% Wasser bezogen auf das Gesamtgewicht der Haarformgebungsrezeptur und des Treibmittels; und
(iv) 1,0 Gew.-% bis 15 Gew.-% Haarformgebungspolymer bezogen auf das Gesamtgewicht der Haarformgebungsrezeptur und des Treibmittels; und
c) eine Sprühvorrichtung, die am Gefäß angebracht ist, um die Haarformgebungsrezeptur aus dem Behälter des Gefäßes abzugeben, und ein Ventil und einen Aktor umfasst, wobei
das Ventil einen Ventilkörper umfasst und der Ventilkörper einen Schaft und ein Federmittel umfasst; und das Ventil ein Stück mit beschränktem Ende (RTP) mit einem Durchmesser von 0,1 mm bis 2,5 mm und einen Dampfphasenhahn (VPT) mit einem Durchmesser von 0,1 mm bis 1,5 mm umfasst;
der Aktor einen Hauptsprühkanal umfasst und mit einem Einsatz kommuniziert, der Einsatz eine Austrittsöffnung und 1 bis 8 Untersprühkanäle umfasst und die Kanäle tangential um die Austrittsöffnung angeordnet sind; und
die Austrittsöffnung in Flüssigkeitskommunikation mit der Haarformgebungsrezeptur im Behälter sein kann;
**dadurch gekennzeichnet, dass** die Austrittsöffnung einen Durchmesser von 0,01 bis 2 mm und eine Öffnungsdicke von 0,1 bis 1,5 mm besitzt; wobei
die Untersprühkanäle eine Höhe von 0,1 bis 0,8 mm und eine Länge von 0,1 bis 0,5 mm besitzen.

2. Aerosolhaarsprayprodukt nach Anspruch 1, das weniger als 20 Gew.-% Alkohol bezogen auf das Gesamtgewicht der Haarformgebungsrezeptur und des Treibmittels umfasst.

3. Aerosolhaarsprayprodukt nach Anspruch 1 oder Anspruch 2, wobei die Rezeptur weniger als 5 Gew.-% Alkohol umfasst.

4. Aerosolhaarsprayprodukt nach einem vorhergehenden Anspruch, wobei das Haarformgebungspolymer in einer Menge von 1,0 bis 10 Gew.-% bezogen auf das Gesamtgewicht der Haarformgebungsrezeptur und des Treibmittels vorliegt.

5. Aerosolhaarsprayprodukt nach einem vorhergehenden Anspruch, wobei das Formgebungspolymer aus einem VA/Crotonate/Vinylneodecanoat-Copolymer, einem Polyurethan-14 (und) AMP-Acrylat-Copolymer, einem Butylester von PVM/MA-Copolymer, Polyester-5, einem Octylacrylamid/Acrylat/Butylaminoethyl-Methacrylat-Copolymer, einem Acrylat/Hydroxyester-Acrylat-Copolymer, Polyurethan-6, einem Acrylat/Octylacrylamid-Copolymer und einem Acrylat-Copolymer und stärker bevorzugt aus einem VA/Crotonat/Vinylneodecanoat-Copolymer und einem Octylacrylamid/Acrylat/Butylaminoethyl-Methacrylat-Copolymer gewählt ist.

6. Aerosolhaarsprayprodukt nach einem vorhergehenden Anspruch, wobei die Rezeptur eine alkalische Basis umfasst.

7. Aerosolhaarsprayprodukt nach einem vorhergehenden Anspruch, wobei das RTP einen Durchmesser von 0,2 bis 1,5 mm und bevorzugt von 0,3 mm bis 1,0 mm besitzt.

8. Aerosolhaarsprayprodukt nach einem vorhergehenden Anspruch, wobei der VPT einen Durchmesser von 0,2 bis 1,0 mm und bevorzugt von 0,3 mm bis 0,8 mm besitzt.

9. Aerosolhaarsprayprodukt nach einem vorhergehenden Anspruch, wobei das Gefäß ein Volumen im Bereich von 200 bis 300 ml besitzt.

10. Aerosolhaarsprayprodukt nach einem vorhergehenden Anspruch, wobei die Austrittsöffnung 6 Untersprühkanäle umfasst.

11. Aerosolhaarsprayprodukt nach einem vorhergehenden Anspruch, wobei die Untersprühkanäle eine Höhe von 0,3 bis 0,7 mm besitzen.

12. Aerosolhaarsprayprodukt nach einem vorhergehenden Anspruch, wobei die Untersprühkanäle eine Länge von 0,25 bis 0,35 mm besitzen.

13. Aerosolhaarsprayprodukt nach einem vorhergehenden Anspruch, wobei die Austrittsöffnung eine Öffnungsdicke von 0,1 bis 1,5 mm besitzt.

14. Verfahren zum Behandeln von Haar mit einem Haarsprayprodukt nach einem der Ansprüche 1 bis 13, das den Schritt des Anwendens einer Haarformgebungsrezeptur nach einem der Ansprüche 1 bis 6 auf das Haar unter Verwendung eines Haarsprayprodukts nach einem der Ansprüche 1 bis 13 umfasst.

## Revendications

1. Produit de laque en aérosol, qui comprend :
a) un récipient pressurisable comprenant
i) une paroi de récipient qui enferme un réservoir pour stocker une formulation pour la coiffure, et
ii) un gaz propulseur ;
b) une formulation pour la coiffure comprenant :
(iii) de 20 % en masse à 75 % en masse, en masse totale de la formulation pour la coiffure et du gaz propulseur ; et
(iv) de 1,0 % en masse à 15 % en masse de polymère pour la coiffure, en masse totale de la formulation pour la coiffure et de gaz propulseur ; et
c) un dispositif de pulvérisation fixé au récipient pour distribuer la formulation pour la coiffure à partir du réservoir du récipient, qui comprend une vanne et un actionneur,
dans lequel la vanne comprend un corps de vanne, ledit corps de vanne comprenant une tige et un moyen de ressort ; et
dans lequel la vanne comprend une pièce à queue étroite (RTP) de diamètre 0,1 mm à 2,5 mm, et un robinet de phase vapeur (VPT) de diamètre 0,1 mm à 1,5 mm ;
dans lequel l'actionneur comprend un canal de pulvérisation principal et communique avec un insert, ledit insert comprenant un orifice de sortie et de 1 à 8 sous-canaux de pulvérisation ; dans lequel lesdits canaux sont tangentiellement disposés autour de l'orifice de sortie ;
dans lequel l'orifice de sortie est capable d'être en communication liquide avec la formulation pour la coiffure dans le réservoir ;
**caractérisé en ce que** l'orifice de sortie présente un diamètre de 0,01 à 2 mm ; et une épaisseur d'orifice de 0,1 à 1,5 mm ;
et dans lequel les sous-canaux de pulvérisation présentent une hauteur de 0,1 à 0,8 mm et une longueur de 0,1 à 0,5 mm.

2. Produit de laque en aérosol selon la revendication 1, qui comprend moins de 20 % en masse d'alcool, en masse totale de la formulation pour la coiffure et de gaz propulseur.

3. Produit de laque en aérosol selon la revendication 1 ou la revendication 2, dans lequel la formulation contient moins de 5 % en masse d'alcool.

4. Produit de laque en aérosol selon l'une quelconque des revendications précédentes, dans lequel le polymère pour la coiffure est présent dans une quantité de 1,0 à 10 % en masse en masse totale de la formulation pour la coiffure et de gaz propulseur.

5. Produit de laque en aérosol selon l'une quelconque des revendications précédentes, dans lequel le polymère pour la coiffure est choisi parmi un copolymère de VA/crotonates/néodécanoate de vinyle, copolymère de polyuréthane-14 (et) AMP-acrylates, ester butylique de copolymère de PVM/MA, polyester-5, copolymère d'octylacrylamide/acrylates/méthacrylate de butylaminoéthyle, copolymère d'acrylates/acrylates d'hydroxyesters, polyuréthane-6, copolymère d'acrylates/octylacrylamide et copolymère d'acrylates, et encore mieux parmi un copolymère de VA/crotonates/néodécanoate de vinyle et copolymère d'octylacrylamide/acrylates/méthacrylate de butylaminoéthyle.

6. Produit de laque en aérosol selon l'une quelconque des revendications précédentes, dans lequel la formulation comprend une base alcaline.

7. Produit de laque en aérosol selon l'une quelconque des revendications précédentes, dans lequel la RTP présente un diamètre de 0,2 à 1,5 mm, de préférence de 0,3 à 1,0 mm.

8. Produit de laque en aérosol selon l'une quelconque des revendications précédentes, dans lequel le VPT présente un diamètre de 0,2 à 1,0 mm, de préférence de 0,3 à 0,8 mm.

9. Produit de laque en aérosol selon l'une quelconque des revendications précédentes, dans lequel le récipient présente un volume de 200 à 300 ml.

10. Produit de laque en aérosol selon l'une quelconque des revendications précédentes, dans lequel l'orifice de sortie comprend 6 sous-canaux de pulvérisation.

11. Produit de laque en aérosol selon l'une quelconque des revendications précédentes, dans lequel les sous-canaux de pulvérisation présentent une hauteur de 0,3 à 0,7 mm.

12. Produit de laque en aérosol selon l'une quelconque des revendications précédentes, dans lequel les sous-canaux de pulvérisation présentent une longueur de 0,25 à 0,35 mm.

13. Produit de laque en aérosol selon l'une quelconque des revendications précédentes, dans lequel l'orifice de sortie présente une épaisseur d'orifice de 0,1 à 1,5 mm.

14. Procédé de traitement des cheveux avec un produit de laque selon l'une quelconque des revendications 1 à 13, comprenant l'étape d'application aux cheveux d'une formulation pour la coiffure des cheveux comme définie dans l'une quelconque des revendications 1 à 6, en utilisant un produit de laque comme défini dans l'une quelconque des revendications 1 à 13.
